# EUROPEAN PATENT APPLICATION

(11) **EP 4 190 811 A1**
(43) Date of publication of application: **07.06.2023**
(21) Application number: 21851315.8
(22) Date of filing: 26.07.2021
(51) Int. Cl.: C07K 16/12, C12N 15/10

(54) **HUMANIZED ANTIBODY SPECIFIC TO BACILLUS ANTHRACIS PROTECTIVE ANTIGEN AND PREPARATION METHOD THEREOF**

(30) Priority: 27.07.2020 KR 20200093382
(71) Applicant: Agency For Defense Development, Daejeon 34060 (KR)
(72) Inventor: YU, Chi-Ho, Daejeon 34060 (KR); KIM, Chang-Hwan, Daejeon 34060 (KR); HUR, Gyeung-Haeng, Daejeon 34060 (KR); JEONG, Seong-Tae, Daejeon 34060 (KR); SONG, Dong-Hyun, Daejeon 34060 (KR); JOE, Hae-Eun, Daejeon 34060 (KR); KIM, Na-Young, Daejeon 34060 (KR); CHOI, Jun-Young, Daejeon 34060 (KR); SHIN, Young-Kee, Daejeon 34060 (KR); JEONG, Hyun-Ho, Daejeon 34060 (KR); JUN, Seung-Chul, Daejeon 34060 (KR)
(74) Representative: Cabinet Laurent & Charras
(86) International application number: PCT/KR2021/009655
(87) International publication number: WO 2022/025571

(57) **Abstract**

The present invention relates to a humanized antibody specific to a *Bacillus anthracis* protective antigen and a preparation method thereof. An antibody library is created by obtaining a *Bacillus anthracis* protective antigen antibody of a monkey from immune cells obtained by immunizing a *Bacillus anthracis* protective antigen, an antibody specific to the *Bacillus anthracis* protective antigen is selected from the created antibody library, and, on the basis of the antibody, a humanized antibody specific to the *Bacillus anthracis* protective antigen is finally selected by creating a humanized antibody library against a *Bacillus anthracis* protective antigen. The selected humanized antibody specific to the *Bacillus anthracis* protective antigen exhibits very high *Bacillus anthracis* protective antigen affinity and toxin neutralization capacity and has a neutralizing effect in the body before and after exposure to *Bacillus anthracis,* and thus can be used as a therapeutic composition for treating or preventing an anthrax.

【Sequence List Text】

<210> 1

<211> 233

<212> PRT

<213> Artificial Sequence

<220>

<223> Light Chain of antibody for anthrax protective antigen

<220>

<221> SIGNAL

<222> (1)..(19)

<220>

<221> SITE

<222> (46)..(51)

<223> CDR1

<220>

<221> SITE

<222> (69)..(71)

<223> CDR2

<220>

<221> SITE

<222> (108)..(116)

<223> CDR3

<400> 1

Met Tyr Leu Gly Leu Asn Tyr Val Phe Ile Val Phe Leu Leu Asn Gly

1               5                   10                  15

Val Gln Ser Asp Ile Gln Met Thr Gln Ser Pro Ser Ser Leu Ser Ala

            20                  25                  30

Ser Val Gly Asp Arg Val Thr Ile Thr Cys Arg Ala Ser Gln Gly Ile

        35                  40                  45

Ser Ser Trp Leu Ala Trp Tyr Gln Gln Lys Pro Gly Lys Ala Pro Lys

    50                  55                  60

Leu Leu Ile Tyr Lys Ala Ser Ser Leu Gln Ser Gly Val Pro Ser Arg

65                  70                  75                  80

Phe Ser Gly Ser Arg Ser Gly Thr Asp Phe Thr Leu Thr Ile Ser Ser

                85                  90                  95

Leu Gln Pro Glu Asp Phe Ala Thr Tyr Tyr Cys Gln His Ser Tyr Gly

            100                 105                 110

Thr Pro Tyr Ser Phe Gly Gln Gly Thr Lys Val Glu Ile Lys Arg Thr

        115                 120                 125

Val Ala Ala Pro Ser Val Phe Ile Phe Pro Pro Ser Asp Glu Gln Leu

    130                 135                 140

Lys Ser Gly Thr Ala Ser Val Val Cys Leu Leu Asn Asn Phe Tyr Pro

145                 150                 155                 160

Arg Glu Ala Lys Val Gln Trp Lys Val Asp Asn Ala Leu Gln Ser Gly

                165                 170                 175

Asn Ser Gln Glu Ser Val Thr Glu Gln Asp Ser Lys Asp Ser Thr Tyr

            180                 185                 190

Ser Leu Ser Ser Thr Leu Thr Leu Ser Lys Ala Asp Tyr Glu Lys His

        195                 200                 205

Lys Val Tyr Ala Cys Glu Val Thr His Gln Gly Leu Ser Ser Pro Val

    210                 215                 220

Thr Lys Ser Phe Asn Arg Gly Glu Cys

225                 230

<210> 2

<211> 468

<212> PRT

<213> Artificial Sequence

<220>

<223> Heavy Chain of antibody for anthrax protective antigen

<220>

<221> SIGNAL

<222> (1)..(19)

<220>

<221> SITE

<222> (45)..(52)

<223> CDR1

<220>

<221> SITE

<222> (70)..(77)

<223> CDR2

<220>

<221> SITE

<222> (116)..(127)

<223> CDR3

<400> 2

Met Tyr Leu Gly Leu Asn Tyr Val Phe Ile Val Phe Leu Leu Asn Gly

1               5                   10                  15

Val Gln Ser Glu Val Gln Leu Val Glu Ser Gly Gly Gly Leu Val Gln

            20                  25                  30

Pro Gly Gly Ser Leu Arg Leu Ser Cys Ala Val Ser Gly Gly Ser Ile

        35                  40                  45

Ser Gly Tyr Trp Trp Gly Trp Ile Arg Gln Ala Pro Gly Lys Gly Leu

    50                  55                  60

Glu Trp Ile Gly Cys Ile Gly Gly Ser Ser Gly Thr Thr Tyr Tyr Asn

65                  70                  75                  80

Pro Ser Leu Lys Ser Arg Val Thr Ile Ser Ala Asp Thr Ser Lys Asn

                85                  90                  95

Thr Ala Tyr Leu Gln Met Asn Ser Leu Arg Ala Glu Asp Thr Ala Val

            100                 105                 110

Tyr Tyr Cys Ala Arg Gly Gly Phe Ser Cys Ser Tyr Tyr Ser Asn Trp

        115                 120                 125

Gly Gln Gly Thr Leu Val Thr Val Ser Ser Ala Ser Thr Lys Gly Pro

    130                 135                 140

Ser Val Phe Pro Leu Ala Pro Ser Ser Lys Ser Thr Ser Gly Gly Thr

145                 150                 155                 160

Ala Ala Leu Gly Cys Leu Val Lys Asp Tyr Phe Pro Glu Pro Val Thr

                165                 170                 175

Val Ser Trp Asn Ser Gly Ala Leu Thr Ser Gly Val His Thr Phe Pro

            180                 185                 190

Ala Val Leu Gln Ser Ser Gly Leu Tyr Ser Leu Ser Ser Val Val Thr

        195                 200                 205

Val Pro Ser Ser Ser Leu Gly Thr Gln Thr Tyr Ile Cys Asn Val Asn

    210                 215                 220

His Lys Pro Ser Asn Thr Lys Val Asp Lys Lys Val Glu Pro Lys Ser

225                 230                 235                 240

Cys Asp Lys Thr His Thr Cys Pro Pro Cys Pro Ala Pro Glu Leu Leu

                245                 250                 255

Gly Gly Pro Ser Val Phe Leu Phe Pro Pro Lys Pro Lys Asp Thr Leu

            260                 265                 270

Met Ile Ser Arg Thr Pro Glu Val Thr Cys Val Val Val Asp Val Ser
        275                 280                 285
His Glu Asp Pro Glu Val Lys Phe Asn Trp Tyr Val Asp Gly Val Glu
    290                 295                 300
Val His Asn Ala Lys Thr Lys Pro Arg Glu Glu Gln Tyr Asn Ser Thr
305                 310                 315                 320
Tyr Arg Val Val Ser Val Leu Thr Val Leu His Gln Asp Trp Leu Asn
                325                 330                 335
Gly Lys Glu Tyr Lys Cys Lys Val Ser Asn Lys Ala Leu Pro Ala Pro
            340                 345                 350
Ile Glu Lys Thr Ile Ser Lys Ala Lys Gly Gln Pro Arg Glu Pro Gln
        355                 360                 365
Val Tyr Thr Leu Pro Pro Ser Arg Asp Glu Leu Thr Lys Asn Gln Val
    370                 375                 380
Ser Leu Thr Cys Leu Val Lys Gly Phe Tyr Pro Ser Asp Ile Ala Val
385                 390                 395                 400
Glu Trp Glu Ser Asn Gly Gln Pro Glu Asn Asn Tyr Lys Thr Thr Pro
                405                 410                 415
Pro Val Leu Asp Ser Asp Gly Ser Phe Phe Leu Tyr Ser Lys Leu Thr
            420                 425                 430
Val Asp Lys Ser Arg Trp Gln Gln Gly Asn Val Phe Ser Cys Ser Val
        435                 440                 445

Met His Glu Ala Leu His Asn His Tyr Thr Gln Lys Ser Leu Ser Leu
    450                 455                 460
Ser Pro Gly Lys
465
<210> 3
<211> 702
<212> DNA
<213> Artificial Sequence
<220>
<223> Light Chain of antibody for anthrax protective antigen
<220>
<221> misc_feature
<222> (136)..(153)
<223> CDR1
<220>
<221> misc_feature
<222> (205)..(213)
<223> CDR2
<220>
<221> misc_feature
<222> (322)..(348)

<223> CDR3

<400> 3

atgtacctgg gcctgaacta cgtgttcatc gtgtttctgc tgaacggcgt gcagtccgac 60

atccagatga cccagtcccc ctcctccctg tccgcctccg tgggcgacag agtgaccatc 120

acctgtcggg cctcccaggg catctcctcc tggctggcct ggtatcagca gaagcccggc 180

aaggccccca agctgctgat ctacaaggcc tcctccctgc agtccggcgt gccctccaga 240

ttctccggct ccagatccgg caccgacttc accctgacca tctcctccct gcagcccgag 300

gacttcgcca cctactactg ccagcactcc tacggcaccc cctactcctt cggccagggc 360

accaaggtgg aaatcaagcg tacggtggcc gctccctccg tgttcatctt cccaccctcc 420

gacgagcagc tgaagtccgg caccgcctcc gtcgtgtgcc tgctgaacaa cttctacccc 480

cgcgaggcca aggtgcagtg gaaggtggac aacgccctgc agtccggcaa ctcccaggaa 540

tccgtcaccg agcaggactc caaggactct acctactccc tgtcctccac cctgaccctg 600

tccaaggccg actacgagaa gcacaaggtg tacgcctgcg aagtgaccca ccagggcctg 660

tcctccccag tgaccaagtc cttcaaccgg ggcgagtgtt ga 702

<210> 4

<211> 1407

<212> DNA

<213> Artificial Sequence

<220>

<223> Heavy Chain of antibody for anthrax protective antigen

<220>

<221> misc_feature

<222> (133)..(156)

<223> CDR1

<220>

<221> misc_feature

<222> (208)..(231)

<223> CDR2

<220>

<221> misc_feature

<222> (346)..(381)

<223> CDR3

<400> 4

atgtacctgg gcctgaacta cgtgttcatc gtgtttctgc tgaacggcgt gcagtccgag 60

gtgcagctgg tggaatctgg cggcggactg gtgcagcctg gcggctccct gagactgtct 120

tgtgccgtgt ccggcggctc catctccggc tattggtggg gctggattcg gcaggctccc 180

ggcaagggcc tggaatggat cggctgcatc ggcggctcct ccggcaccac ctactacaac 240

ccctccctga agtccagagt gaccatctcc gccgacacct ccaagaacac cgcctacctg 300

cagatgaact ccctgcgggc cgaggacacc gccgtgtact actgtgccag aggcggcttc 360

tcctgctcct actactccaa ctggggccag ggcaccctgg tcaccgtgtc ctccgcctcc 420

accaagggcc cctccgtgtt ccctctggcc ccatcctcca agtccacctc tggcggcacc 480

gccgctctgg gctgcctggt caaggactac ttccccgaac cggtgacagt gtcctggaac 540

tctggcgccc tgacctccgg cgtgcacacc ttccctgccg tgctgcagtc ctccggcctg 600

tactccctgt cctccgtcgt gaccgtgccc tcatcctccc tgggcaccca gacctacatc 660

tgcaacgtga accacaagcc ctccaacacc aaggtggaca agaaggtgga acccaagtcc 720

tgcgacaaga cccacacctg tcccccctgc cctgcccctg aactgctggg cggaccttcc 780

gtgttcctgt tccccccaaa gcccaaggac accctgatga tctcccggac ccccgaagtg 840

acctgcgtgg tggtggacgt gtcccacgag gaccctgaag tgaagtttaa ttggtacgtg 900

gacggcgtgg aagtgcacaa cgccaagacc aagcccagag aggaacagta caactccacc 960

taccgggtgg tgtccgtgct gacagtgctg caccaggact ggctgaacgg caaagagtac 1020

aagtgcaagg tgtccaacaa ggccctgcct gcccccatcg aaaagaccat ctccaaggct 1080

aagggccagc cccgcgagcc ccaggtgtac accctgcctc catctcggga cgagctgacc 1140

aagaaccagg tgtccctgac atgtctggtc aagggcttct acccctccga tatcgccgtg 1200

gaatgggagt ccaacggcca gcccgagaac aactacaaga ccaccccccc tgtgctggac 1260

tccgacggct cattcttcct gtactccaag ctgaccgtgg acaagtcccg gtggcagcag 1320

ggcaacgtgt tctcctgctc cgtgatgcac gaggccctgc acaaccacta cacccagaag 1380

tccctgtccc tgtctcccgg caagtga 1407

<210> 5

<211> 6

<212> PRT

<213> Artificial Sequence

<220>

<223> Light Chain CDR1

<400> 5

Gln Gly Ile Ser Ser Trp
1               5

<210> 6
<211> 3
<212> PRT
<213> Artificial Sequence
<220>
<223> Light Chain CDR2
<400> 6
Lys Ala Ser
1

<210> 7
<211> 9
<212> PRT
<213> Artificial Sequence
<220>
<223> Light Chain CDR3
<400> 7
Gln His Ser Tyr Gly Thr Pro Tyr Ser
1               5

<210> 8

<211> 8

<212> PRT

<213> Artificial Sequence

<220>

<223> Heavy Chain CDR1

<400> 8

Gly Gly Ser Ile Ser Gly Tyr Trp

1               5

<210> 9

<211> 8

<212> PRT

<213> Artificial Sequence

<220>

<223> Heavy Chain CDR2

<400> 9

Ile Gly Gly Ser Ser Gly Thr Thr

1               5

<210> 10

<211> 12

<212> PRT

<213> Artificial Sequence

<220>

<223> Heavy Chain CDR3

<400> 10

Ala Arg Gly Gly Phe Ser Cys Ser Tyr Tyr Ser Asn

1               5                   10

<210> 11

<211> 18

<212> DNA

<213> Artificial Sequence

<220>

<223> Light Chain CDR1

<400> 11

cagggcatct cctcctgg 18

<210> 12

<211> 9

<212> DNA

<213> Artificial Sequence

<220>

<223> Light Chain CDR2

<400> 12

aaggcctcc 9

<210> 13

<211> 27

<212> DNA

<213> Artificial Sequence

<220>

<223> Light Chain CDR3

<400> 13

cagcactcct acggcacccc ctactcc 27

<210> 14

<211> 24

<212> DNA

<213> Artificial Sequence

<220>

<223> Heavy Chain CDR1

<400> 14

ggcggctcca tctccggcta ttgg 24

<210> 15

<211> 24

<212> DNA

<213> Artificial Sequence

<220>

<223> Heavy Chain CDR2

<400> 15

atcggcggct cctccggcac cacc 24

<210> 16

<211> 36

<212> DNA

<213> Artificial Sequence

<220>

<223> Heavy Chain CDR3

<400> 16

gccagaggcg gcttctcctg ctcctactac tccaac 36

## Description

### [Technical Field]

The present invention relates to a monoclonal humanized antibody that specifically binds to a Bacillus anthracis protective antigen, which can treat Bacillus anthracis infection, and relates to a humanized antibody specific to a Bacillus anthracis protective antigen and a method for preparing the same.

### [Background Art]

Bacillus anthracis, the causative agent of anthrax, is a gram-positive Bacillus and forms spores with high resistance in harsh environments. Spores are non-productive bacterial forms in a dormant state and have high resistance to environmental conditions such as UV rays, dryness, and high temperatures. Spores can survive for decades in nature and are very difficult to be eliminated. Anthrax most commonly occurs in herbivores, including livestock, and people who are exposed to infected livestock or ingest products made from infected livestock have also been reported to get anthrax. Bacillus anthracis has been classified by the American CDC (Centers for disease control and prevention) as a pathogenic microorganism of Category A, which has high potential for use in biological warfare or biological terrorism.

In humans, Bacillus anthracis infection occurs when spores enter the host by one of three routes: a dermal route through cuts and abrasions of the skin, a gastrointestinal route through consumption of contaminated meat, and an inhalation route through inhalation of airborne spores. Bacillus anthracis infection due to the inhalation route is the most lethal form of disease because of a difficulty in timely diagnosis of inhaled Bacillus anthracis and a potential to affect large numbers of people if deliberately distributed. After inhalation, Bacillus anthracis spores are phagocytosed by alveolar macrophages and migrate to mediastinum nodes. During this process, Bacillus anthracis germinates into vegetative cells and enters the blood, causing sepsis. The incubation period for the disease is usually 1 to 6 days and begins with flu-like symptoms such as fatigue and slightly elevated body temperature. These symptoms may develop into symptoms such as respiratory failure followed by rapid respiratory failure and shock even when intensive antibiotic treatment is administered.

Bacillus anthracis has two virulence factors, a capsule that evades the bacterium from phagocytosis, and two exotoxins, lethal toxin and edema toxin, that are involved in the pathogenesis of anthrax. Bacillus anthracis secretes anthrax toxin through the pXO1 plasmid, which consists of three types of toxin proteins: a protective antigen with a molecular weight of 83 kDa, a lethal factor with a molecular weight of 90 kDa, and an edema factor with a molecular weight of 89 kDa.

The protective antigen forms a cell binding molecule for a lethal factor and an edema factor to enter the cell. The lethal toxin is a zinc protease that blocks signaling by cleaving various mitogen activated protein kinase kinases. The edema factor is a calmodulin-dependent adenylate cyclase that produces abnormally high amounts of cAMP. As a result, phagocytosis by macrophages is inhibited and water homeostasis is disrupted, resulting in edema.

To date, several antibiotics, including ciprofloxacin and doxycycline, have been mainly used for the treatment of Bacillus anthracis. These antibiotics are known to show very effective therapeutic effects when administered immediately after exposure, but the initial symptoms of anthrax are very similar to those of the common cold, making it very difficult to diagnose and treat the disease in the early stages. In addition, it is difficult to expect a therapeutic effect on antibiotic-resistant strains that can be used in biochemical terrorism or biological warfare. In various experimental models, passive immunization using antibodies has been reported to be very effective in protecting against anthrax, suggesting that antibodies play an important role in protecting against anthrax (Huang et al. 2015 toxin 7(10): 3960-3976, Krishnan 2015 Clin vaccine immunol. 22(4): 430-439).

As a Bacillus anthracis vaccine, there are the US-approved anthrax vaccine adsorbed (AVA, also known as 'BioThraxⓡ') vaccine and the UK-approved anthrax vaccine precipitated (AVP) vaccine. The protective effect of these vaccines is based on the induction of an antibody response to a Bacillus anthracis antigen, which is the main component of the protective antigen. However, these vaccines have not been fully tested for safety, so they are limited to military personnel or some people at high risk of exposure to the Bacillus anthracis. In addition, the repetitive administration of vaccines and a period of several months are required to acquire immunity capable of defense, making it virtually impossible to use the vaccines in an emergency. Therefore, there is an urgent need to develop an antibody therapeutic agent capable of efficiently preventing and treating anthrax by neutralizing the Bacillus anthracis antigen quickly and effectively.

### [Disclosure]

### [Technical Problem]

Considering the above matters, an object of the present invention is an effective treatment using an antibody based on a recombinant antigen in consideration of human safety, and to provide a humanized antibody specific to the Bacillus anthracis protective antigen as an antibody that specifically binds to the Bacillus anthracis protective antigen, which is a monoclonal humanized antibody that can bind to the Bacillus anthracis protective antigen with specificity and affinity, and a method for preparing the same.

Another object of the present invention is to provide a method for preventing or treating an anthrax using a humanized antibody specific to a Bacillus anthracis protective antigen.

### [Technical Solution]

In order to achieve the above object, a humanized antibody specific to a Bacillus anthracis protective antigen includes a light chain consisting of an amino acid sequence represented by SEQ ID NO: 1, and a heavy chain consisting of an amino acid sequence represented by SEQ ID NO: 2.

In the humanized antibody specific to a Bacillus anthracis protective antigen, a nucleic acid encoding the light chain may be represented by a nucleotide sequence of SEQ ID NO: 3.

The light chain may include a light chain CDR1 represented by an amino acid sequence of SEQ ID NO: 5, a light chain CDR2 represented by an amino acid sequence of SEQ ID NO: 6, and a light chain CDR3 represented by an amino acid sequence of SEQ ID NO: 7.

In the humanized antibody specific to a Bacillus anthracis protective antigen, reviewing the nucleotide sequences encoding the light chain CDR1, light chain CDR2, and light chain CDR3 constituting the light chain, the light chain CDR1 may be encoded by a nucleotide sequence of SEQ ID NO: 11, the light chain CDR2 may be encoded by a nucleotide sequence of SEQ ID NO: 12, and the light chain CDR3 may be encoded by a nucleotide sequence of SEQ ID NO: 13.

In the humanized antibody specific to a Bacillus anthracis protective antigen, a nucleic acid encoding the heavy chain may be represented by a nucleotide sequence of SEQ ID NO: 4.

The heavy chain may include a heavy chain CDR1 represented by an amino acid sequence of SEQ ID NO: 8, a heavy chain CDR2 represented by an amino acid sequence of SEQ ID NO: 9, and a heavy chain CDR3 represented by an amino acid sequence of SEQ ID NO: 10.

In the humanized antibody specific to a Bacillus anthracis protective antigen, reviewing the nucleotide sequences encoding the heavy chain CDR1, heavy chain CDR2, and heavy chain CDR3 constituting the heavy chain, the heavy chain CDR1 may be encoded by a nucleotide sequence of SEQ ID NO: 14, the heavy chain CDR2 may be encoded by a nucleotide sequence of SEQ ID NO: 15, and the heavy chain CDR3 may be encoded by a nucleotide sequence of SEQ ID NO: 16.

In addition, in order to achieve the above object, a method for preparing a humanized antibody specific to a Bacillus anthracis protective antigen, may include (a) preparing an antibody library by synthesizing an antibody to a Bacillus anthracis protective antigen, (b) selecting a Bacillus anthracis protective antigen antibody clone as an antibody that specifically binds to the Bacillus anthracis protective antigen from the antibody library, (c) expressing and purifying a Bacillus anthracis protective antigen monoclonal antibody by introducing an expression vector containing a gene of the Bacillus anthracis protective antigen antibody clone selected in step (b) into an antibody expressing host cell, (d) preparing a humanized antibody library of the Bacillus anthracis protective antigen containing complementarity-determining regions of an antibody using the Bacillus anthracis protective antigen monoclonal antibody as a template, (e) selecting a humanized antibody clone of the Bacillus anthracis protective antigen as an antibody specifically binding to the Bacillus anthracis protective antigen from the humanized antibody library of the Bacillus anthracis protective antigen, and (f) preparing a humanized antibody specific to the Bacillus anthracis protective antigen by introducing an animal cell expression vector containing the gene of the humanized antibody clone of the Bacillus anthracis protective antigen selected through step (e) into the antibody expressing host cell.

In the method for preparing a humanized antibody specific to a Bacillus anthracis protective antigen, the (c) step may express and purify the Bacillus anthracis protective antigen monoclonal antibody by including the steps of preparing a recombinant expression vector containing the gene of the Bacillus anthracis protective antigen antibody clone, introducing the gene of the Bacillus anthracis protective antigen antibody clone into the antibody expressing host cell using the recombinant expression vector and transforming the host cell, and purifying the antibody from a culture medium in which the transformed antibody expressing host cell is cultured.

In the method for preparing a humanized antibody specific to a Bacillus anthracis protective antigen, the (f) step may prepare a final humanized antibody specific to the Bacillus anthracis protective antigen by including the steps of preparing a recombinant animal cell expression vector containing the gene of the humanized antibody clone of the Bacillus anthracis protective antigen from the humanized antibody library of the Bacillus anthracis protective antigen, introducing the gene of the humanized antibody clone of the Bacillus anthracis protective antigen into the antibody expressing host cell using the recombinant animal cell expression vector and transforming the host cell, and purifying the humanized antibody specific to the Bacillus anthracis protective antigen from a culture medium in which the transformed antibody expressing host cell is cultured.

In the (d) step, in order to prepare a humanized antibody specific to a Bacillus anthracis protective antigen, in case that a complementarity-determining region of the antibody is a light chain complementarity-determining region of the antibody, the light chain amino acid sequence of the antibody may include a light chain CDR1 represented by an amino acid sequence of SEQ ID NO: 5, a light chain CDR2 represented by an amino acid sequence of SEQ ID NO: 6, and a light chain CDR3 represented by an amino acid sequence of SEQ ID NO: 7.

As the light chain complementarity-determining region, the light chain CDR1 may be encoded by a nucleotide sequence of SEQ ID NO: 11, the light chain CDR2 may be encoded by a nucleotide sequence of SEQ ID NO: 12, and the light chain CDR3 may be encoded by a nucleotide sequence of SEQ ID NO: 13.

In the (d) step, in order to prepare a humanized antibody specific to a *Bacillus anthracis* protective antigen, in case that a complementarity-determining region of the antibody is a heavy chain complementarity-determining region of the antibody, the heavy chain amino acid sequence of the antibody may include a heavy chain CDR1 represented by an amino acid sequence of SEQ ID NO: 8, a heavy chain CDR2 represented by an amino acid sequence of SEQ ID NO: 9, and a heavy chain CDR3 represented by an amino acid sequence of SEQ ID NO: 10.

As the heavy chain complementarity-determining region, the heavy chain CDR1 may be encoded by a nucleotide sequence of SEQ ID NO: 14, the heavy chain CDR2 may be encoded by a nucleotide sequence of SEQ ID NO: 15, and the heavy chain CDR3 may be encoded by a nucleotide sequence of SEQ ID NO: 16.

The humanized antibody specific to the *Bacillus anthracis* protective antigen prepared by the method for preparing the humanized antibody specific to the *Bacillus anthracis* protective antigen includes a light chain consisting of an amino acid sequence represented by SEQ ID NO: 1, and a heavy chain consisting of an amino acid sequence represented by SEQ ID NO: 2.

In order to achieve another object, the present invention provides a preventive and therapeutic composition that can be effectively used for the treatment of anthrax, a disease caused by a Bacillus anthracis infection, including as an active ingredient a humanized antibody specific to the Bacillus anthracis protective antigen.

The humanized antibody specific to the Bacillus anthracis protective antigen included in the composition for preventing and treating a disease caused by the Bacillus anthracis infection has high specific affinity for the Bacillus anthracis protective antigen and excellent ability to neutralize anthrax toxin. Thus, it is a pharmaceutical composition that can be effectively used in the treatment of diseases caused by the Bacillus anthracis infection.

### [Advantageous Effects]

The humanized antibody specific to a Bacillus anthracis protective antigen according to the present invention has very high affinity with the Bacillus anthracis protective antigen and excellent toxin neutralization capability, and immediately induces high protective efficacy before and after exposure to the Bacillus anthracis in vivo, thereby exhibiting effective toxin neutralization capability of the Bacillus anthracis. Unlike general immunization, which requires raising an immune response and maintaining immunity through booster vaccination, it has an effect of effectively treating or preventing diseases caused by the Bacillus anthracis with just one administration.

### [Description of Drawings]

FIG. 1 is a flowchart of a method for preparing a humanized antibody specific to a Bacillus anthracis protective antigen according to an embodiment of the present invention.
FIG. 2 is a result of analyzing a binding ability of an expressed scFv-Fc antibody against a Bacillus anthracis protective antigen.
FIGS. 3 and 4 show results of Biacore analyses on an antigen binding affinity of a humanized antibody specific to a Bacillus anthracis protective antigen.
FIG. 5 shows an anthrax toxin-neutralization capability of a purified antibody of a Bacillus anthracis protective antigen analyzed through a neutralization capability test using mouse macrophage cells.
FIG. 6 shows an evaluation of a therapeutic effect of an antibody by administering a lethal factor and a humanized antibody of a protective antigen to rats.
FIG. 7 shows an evaluation of a therapeutic effect of an antibody by administering a humanized antibody of a protective antigen and a Bacillus anthracis to rabbits.

### [Mode for Invention]

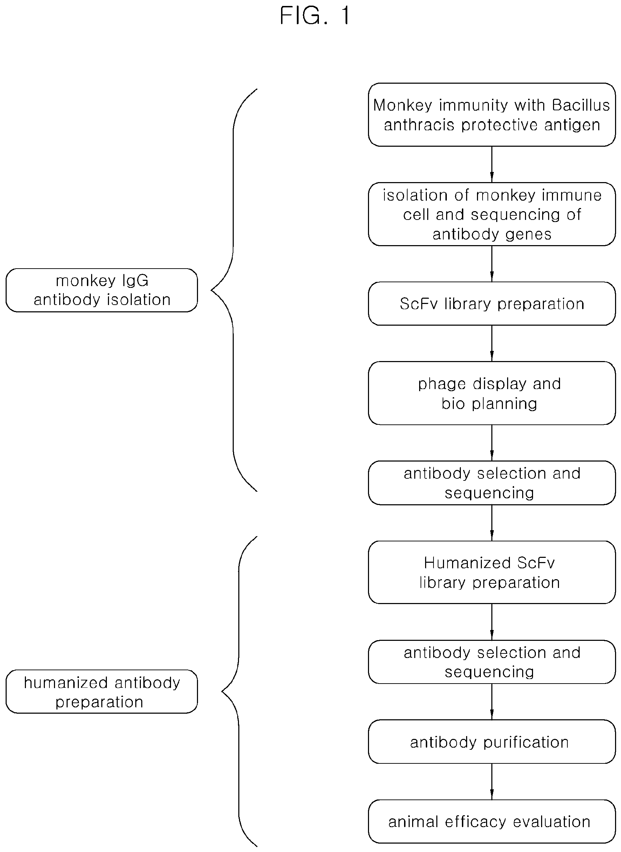

FIG. 1 is a flowchart of a method for preparing a humanized antibody specific to a Bacillus anthracis protective antigen according to an embodiment of the present invention.

As shown in FIG. 1, a method for preparing a humanized antibody specific to a Bacillus anthracis protective antigen largely includes a step of isolating a monkey IgG antibody and a step of preparing a humanized antibody. An antibody library (scFv library) was prepared by obtaining monkey antibodies from immune cells obtained by immunizing monkeys with Bacillus anthracis protective antigens (PAs), and then antibodies specific to the Bacillus anthracis protective antigens were selected through phage display and bio panning. In order to prepare a humanized antibody from the selected antibodies, a humanized antibody library (scFv library) was prepared and a humanized antibody specific to the Bacillus anthracis protective antigen was selected. The animal efficacy evaluation of the selected antibody was conducted to evaluate the efficacy of the antibody.

Hereinafter, an antibody specific to a Bacillus anthracis protective antigen and a method for preparing an antibody specific to a Bacillus anthracis protective antigen of the present invention will be described in more detail through examples.

### Example 1: Preparation of a monkey antibody library of a Bacillus anthracis protective antigen

Example 1 describes the preparation of a monkey single-chain variable fragment (scFv) library against a Bacillus anthracis protective antigen.

The term "antibody" refers to a protein specifically binding to a target antigen, and encompasses both of a polyclonal antibody and a monoclonal antibody. In addition, the term is intended to encompass any forms produced by genetic engineering, such as chimeric antibodies and heterogeneous antibodies. Especially, the antibody may be, but is not limited to, a heterotetramer consisting of two light chains and two heavy chains, while each of the chains may include a variable domain having a variable amino acid sequence and a constant domain having a constant amino acid sequence.

The term "single-chain variable fragment (scFv)" refers to a protein in which light chain and heavy chain variable domains of an antibody are linked to each other via a linker including a peptide chain of 15 or less amino acids connected. The scFv may have both an order of light chain variable domain-linker-heavy chain variable domain and an order of heavy chain variable domain-linker-light chain variable domain, are has the same or similar antigen specificity compared with its original antibody. In addition, the term "antibody library" refers to a collection of various antibody genes having different sequences.

### 1-1. Preparation of a monkey scFv cDNA library against a Bacillus anthracis protective antigen

Immune cells are obtained from a monkey immunized with a Bacillus anthracis protective antigen, RNAs of the immune cells are isolated, and cDNA for the Fd region of the light chain and heavy chain of the Bacillus anthracis protective antigen antibody of the monkey is synthesized from the isolated RNAs.

Specifically, blood was taken from two monkeys immunized by injecting the Bacillus anthracis protective antigen, lymphocytes were separated as the immune cells using a Ficoll concentration gradient, and all RNAs were isolated from the isolated lymphocytes (RNAzol, Sigma). Using these as a template, cDNAs were synthesized using reverse transcriptase (Superscript II, ThermoFisher Scientific) and Oligo dT20 primer (ThermoFisher Scientific).

Next, the monkey scFv DNA fragments of the Bacillus anthracis protective antigen for use in a monkey single-chain fragment antibody (scFv) library of the Bacillus anthracis protective antigen were amplified by performing overlap extension PCR on the heavy and light chains of the Bacillus anthracis protective antigen antibody of the monkey using the synthesized cDNAs as a template. In this case, for the primers for amplifying the heavy chain, linker, and light chain of the Bacillus anthracis protective antigen antibody of the monkey, the 5'-specific heavy chain primer, linker primer, and light chain primer of the heavy chain variable domain of the Bacillus anthracis protective antigen antibody of the monkey were designed by analyzing the monkey antibody gene sequence obtained from monkey antibody database (http://www.kcl.ac.uk/immunobiology/Mac_ig/). The primer sequences were summarized in Tables 1 and 2 below.

Table 1 shows the primer sequences used for the synthesis of the monkey single-chain variable fragment (scFv) of the *Bacillus anthracis* protective antigen in Example 1.

**[Table 1]**

| Category | | Name | Sequence (5'-3') |
|---|---|---|---|
| Heavy chain | F | 23HSCVH1-FL | CAGGTGCAGCTGGTGCAGTCTGG |
| | | 23HSCVH2-FL | CAGATCACCTTGAAGGAGTCTGG |
| | | 23HSCVH3F | GAGGTGCAGCTGGTGSAGTCTGG |
| | | 23HSCVH3a-FL | GAGGTGCAGCTGKTGGAGTCTGG |
| | | 23HSCVH4-FL | CAGGTGCAGCTGCAGGAGTCGGG |
| | | 23HSCVH4a-FL | CAGGTGCAGCTACAGCAGTGGGG |
| | R | MonJHr1 | CCTGGCCGGCCTGGCCTGAGGAGACGGTGAC CAGAAC |
| | | MonJHr2 | CCTGGCCGGCCTGGCCTGAGGAGACGGTGAC SASGACC |
| | | MonJHr3 | CCTGGCCGGCCTGGCCTGAGGAGATGGTGAT YGGGGTGC |
| Kappa | F | HSCG1234-B | CCTGGCCGGCCTGGCCACTAGTGACCGATGG GCCCTTGGTGGARGC |
| | | HSCM-B | CCTGGCCGGCCTGGCCACTAGTAAGGGTTGG GGCGGATGCACTCCC |
| | | MonIgKv1f | GGGCCCAGGCGGCCGACATYCAGATGACCCA GTCTC |
| | | MonIgkv2f_a | GGGCCCAGGCGGCCGATGTTGTRATGACTCA GTCTCC |
| | | MonIgkv2_b_C | GGGCCCAGGCGGCCGATAYTGTGATGAYCCA GACTC |
| | R | MonIgKv3f_a_b | GGGCCCAGGCGGCCGAAATWGTRATGACGC AGTCTCC |
| | | MonIgKv3f_c | GGGCCCAGGCGGCCCAAGTTATATTGACTCA GTCTCCAG |
| Lamb da | F | MonC_Kappa_r | GACAGATGGTGSAGCCAC |
| | | MonIgLv1ps_a | GGGCCCAGGCGGCCCAGTCTGTGCTGACGCA GCC |
| | | MonIgLv1ps_b | GGGCCCAGGCGGCCCAGTCTGTACTGACTCA GCTGC |
| | | MonIgLv2f_a | GGGCCCAGGCGGCCCAGGCTGCCCCGACTCA G |
| | | MonIgLv2f_b | GGGCCCAGGCGGCCCAGTCTGCCCCGAYTCA GYC |
| | | MonIgLv2fd | GGGCCCAGGCGGCCCAGGCTGCCCTGACTCA GC |
| | | MonIglv3f_a | GGGCCCAGGCGGCCTCCTCTGRGCTGACTCA GRAG |
| | | MonIglv3f_b | GGGCCCAGGCGGCCTCTTCTGAGCTGACTCA GGACC |
| | | MonIglv3f_c | GGGCCCAGGCGGCCTCCTATGAGCTGACACA GCCAC |
| | | MonkIlv4 | GGGCCCAGGCGGCCCAGCCTGTGCTGACTCA GTCG |
| | | MonIglV5fa | GGGCCCAGGCGGCCAAGCCTATGCTGACTCA GCCG |
| | R | MonIglV5fb | GGGCCCAGGCGGCCCAGCCTGTGCTGACYCA GCC |
| | | MonC_lamda_r | GGGGGCAGCCTTKGGYTG |
| Linker | F | RLc_KF | GTGGCTSCACCATCTGTCGGTGGTTCCTCTAG ATCT |
| | | RLc_LF | CARCCMAAGGCTGCCCCCGGTGGTTCCTCTA GATCT |
| | R | RHSCVH1-FL | CCAGACTGCACCAGCTGCACCTGCCCA |
| | | RHSCVH2-FL | CCAGACTCCTTCAAGGTGATCTGCCCACCAC CGCCCGA |
| | | RHSCVH3-FL | CCAGACTSCACCAGCTGCACCTCCCCACCAC C |
| | | RHSCVH3a-FL | CCAGACTCCAMCAGCTGCACCTCCCCACCAC CGCCCGA |
| | | RHSCVH4-FL | CCCGACTCCTGCAGCTGCACCTGCCCACCA |
| | | RHSCVH4a-FL | CCCCACTGCTGTAGCTGCACCTGCCCA |
| Ampli fication | F | RSC-Fa | GAGGAGGAGGAGGAGGAGGCGGGGCCCAGG CGGCCGAH |
| | | RSC-Fb | GAGGAGGAGGAGGAGGAGGCGGGGCCCAGG CGGCCCAGB |
| | | RSC-Fc | GAGGAGGAGGAGGAGGAGGCGGGGCCCAGG CGGCCTCYT |
| | R | RSC-B2 | GAGGAGGAGGAGGAGGAGCCTGGCCGGCCT GGCCTGAGGAG |
| | | RSC-B | GAGGAGGAGGAGGAGGAGCCTGGCCGGCCT GGCCACTAGTG |

Table 2 is a table showing primer combinations shown in Table 1 for performing PCR in Example 1.

**[Table 2]**

| Category | PCR No. | Primer |
|---|---|---|
| Heavy chain | 1 | 23HSCVH1-FL, MonJHr1 |
| | 2 | 23HSCVH2-FL, MonJHr1 |
| | 3 | 23HSCVH3FL, MonJHr1 |
| | 4 | 23HSCVH3a-FL, MonJHr1 |
| | 5 | 23HSCVH4-FL, MonJHr1 |
| | 6 | 23HSCVH4a-FL, MonJHr1 |
| | 7 | 23HSCVH1-FL, MonJHr2 |
| | 8 | 23HSCVH2-FL, MonJHr2 |
| | 9 | 23HSCVH3FL, MonJHr2 |
| | 10 | 23HSCVH3a-FL, MonJHr2 |
| | 11 | 23HSCVH4-FL, MonJHr2 |
| | 12 | 23HSCVH4a-FL, MonJHr2 |
| | 13 | 23HSCVH1-FL, MonJHr3 |
| | 14 | 23HSCVH2-FL, MonJHr3 |
| | 15 | 23HSCVH3FL, MonJHr3 |
| | 16 | 23HSCVH3a-FL, MonJHr3 |
| | 17 | 23HSCVH4-FL, MonJHr3 |
| | 18 | 23HSCVH4a-FL, MonJHr3 |
| | 19 | 23HSCVH1-FL, HSCG1234-B |
| | 20 | 23HSCVH2-FL, HSCG1234-B |
| | 21 | 23HSCVH3FL, HSCG1234-B |
| | 22 | 23HSCVH3a-FL, HSCG1234-B |
| | 23 | 23HSCVH4-FL, HSCG1234-B |
| | 24 | 23HSCVH4a-FL, HSCG1234-B |
| | 25 | 23HSCVH1-FL, HSCM-B |
| | 26 | 23HSCVH2-FL, HSCM-B |
| | 27 | 23HSCVH3FL, HSCM-B |
| | 28 | 23HSCVH3a-FL, HSCM-B |
| | 29 | 23HSCVH4-FL, HSCM-B |
| | 30 | 23HSCVH4a-FL, HSCM-B |
| Kappa chain | 1 | MonIgKv1f, MonC_Kappa_r |
| | 2 | Monlgkv2f_a, MonC_Kappa_r |
| | 3 | MonIgkv2_b_C, MonC_Kappa_r |
| | 4 | MonIgKv3f_a_b, MonC_Kappa_r |
| | 5 | MonIgKv3f_c, MonC_Kappa_r |
| Lambda chain | 1 | MonIgLv1ps_a, MonC_lamda_r |
| | 2 | MonIgLv1ps_b, MonC_lamda_r |
| | 3 | MonIgLv2f_a, MonC_lamda_r |
| | 4 | MonIgLv2f_b, MonC_lamda_r |
| | 5 | MonIgLv2fd, MonC_lamda_r |
| | 6 | MonIglv3f_a, MonC_lamda_r |
| | 7 | MonIglv3f_b, MonC_lamda_r |
| | 8 | MonIglv3f_c, MonC_lamda_r |
| | 9 | MonkIlv4, MonC_lamda_r |
| | 10 | MonIglV5fa, MonC_lamda_r |
| | 11 | MonIglV5fb, MonC_lamda_r |
| Linker (kappa) | 1 | RHSCVH1-FL, RLc_KF |
| | 2 | RHSCVH2-FL, RLc_KF |
| | 3 | RHSCVH3-FL, RLc_KF |
| | 4 | RHSCVH3a-FL, RLc_KF |
| | 5 | RHSCVH4-FL, RLc_KF |
| | 6 | RHSCVH4a-FL, RLc_KF |
| Linker (Lambda) | 7 | RHSCVH1-FL, RLc_LF |
| | 8 | RHSCVH2-FL, RLc_LF |
| | 9 | RHSCVH3-FL, RLc_LF |
| | 10 | RHSCVH3a-FL, RLc_LF |
| | 11 | RHSCVH4-FL, RLc_LF |
| | 12 | RHSCVH4a-FL, RLc_LF |
| Amplification | 1 | RSC-Fa, RSC-B |
| | 2 | RSC-Fa, RSC-B2 |
| | 3 | RSC-Fb, RSC-B |
| | 4 | RSC-Fc, RSC-B |

As shown in Table 2, for the heavy chain variable domain of an antibody, the cDNA library of the Fd region of the protective antigen antibody of the monkey was selectively synthesized by PCR using the 5' specific primers of the heavy chain variable domain, 23HSCVH1-FL, 23HSCVH2-FL, 23HSCVH3-FL, 23HSCVH3a-FL, 23HSCVH4-FL, 23HSCVH4a-FL, and the 3' specific primers of the heavy chain variable domain, MonJHr1, MonJHr2, MonJHr3, HSCG1234- B, HSCM-B as a pair, respectively, and the synthesized cDNA as a template.

In addition, for the light chain variable domain of an antibody, the light chain cDNA library of the Bacillus anthracis protective antigen antibody of the monkey was selectively synthesized and prepared by PCR using the 5' specific primers, MonlgKv1f, MonlgKv2f_b_c, MonlgKv3f_a_b, and MonC_Kappa_r primers as a pair, in the case of the Kappa variable domain of the light chain, and the 5' specific primers, MonlgKv1ps_a, MonlgKv1ps_b, MonlgKv2f_a, MonlgKv2f_b, MonlgKv3f_a, MonlgKv3f_b, MonlgKv3f_c, MonlgKv4, MonlgKv5fa, MonlgKv5fb, and MonC_labda_r primers as a pair, in the case of Lambda, respectively, and the cDNA as a temperate.

In addition, for the linker, the linker cDNA library of the Bacillus anthracis protective antigen antibody of the monkey was selectively synthesized and prepared by PCR using the 5' specific primers, RLc_KF and RLc-LF, and the 3' specific primers, RHSCVH1-FL, RHSCVH2-FL, RHSCVH3-FL, RHSCVH3a-FL, RHSCVH4-FL, and RHSCVH4a-FL.

The PCR reaction for the heavy chain variable domain, light chain variable domain, and linker of the Bacillus anthracis protective antigen antibody of the monkey was performed 30 times at 95 °C for 40 seconds, at 60 °C for 40 seconds, and at 72 °C for 1 minute after by pre-denaturation at 95 °C for 5 minutes, using a Taq DNA polymerase.

In addition, in order to increase the cloning efficiency of the cDNA library for the heavy chain, light chain, and linker of the Bacillus anthracis protective antigen antibody of the monkey, the PCR was preformed using the 5' primers, RSC-Fa, RSC-Fb, RSC-Fc primers, and the 3' primers, RSC-B2, RSC-B PCR primers. The PCR reaction was performed 30 times at 95 °C for 50 seconds, 60 °C for 40 seconds, and 72 °C for 1 minute after pre-denaturation at 95 °C for 5 minutes, using the Taq DNA polymerase.

### 1-2. Cloning and transformation

In order to prepare a single-chain variable fragment (scFv) library for the Bacillus anthracis protective antigen of the monkey, the gene fragments of the Bacillus anthracis protective antigen antibody of the monkey from the scFv cDNA library obtained above were cloned into a pComb3xss vector and transformed into host cells, E. coli, to prepare the scFv library for the Bacillus anthracis protective antigen of the monkey.

Specifically, the scFv DNA fragments, which are the genes of the Bacillus anthracis protective antigen antibody of the monkey obtained from the PCR, and the pComb3bss vector were each digested with a restriction enzyme, Sfi I, purified, and then, the two cut DNA fragments were left at 16 °C overnight and ligated, and then the enzyme was inactivated by heating at 70 °C for 10 minutes. Thereafter, the scFv library plasmid was purified using a PCR purification kit, and the scFv library plasmid was transformed into E. coli ER2738 by electroporation to prepare the scFv library for the Bacillus anthracis protective antigen of the monkey of 1×10⁹ in size.

### Example 2: Selection of an antibody that specifically binds to the Bacillus anthracis protective antigen

In order to select an antibody specifically binding to the Bacillus anthracis protective antigen, a panning using a phage display method was performed using the scFv library of the Bacillus anthracis protective antigen of the monkey according to above Example 1.

The term "phage display", as used herein, refers to a method in which the gene of M13 bacteriophage is manipulated, the gene of a foreign protein is fused to one gene of the surface protein of the bacteriophage, and the foreign protein is fused to the surface protein of the produced phage and displayed on the surface of the phage. When a protein is presented on the surface, a foreign gene is often fused to the 5' site of a gIII gene.

The term "panning", as used herein, refers to a process of selectively amplifying only clone that binds to a specific molecule from a protein library, such as an antibody displayed on a phage surface. A phage library is added to the target molecule immobilized on the surface to induce binding, non-bound phage clones are removed by washing, and then, only the bound phage clones are eluted to infect the E. coli host again, and targetbound phage clones are subject to the process of amplifying them using a helper phage. In most cases, this process is repeated 3 to 4 times or more to maximize the ratio of binding clones.

### 2-1. Bio-panning using phage display

In the phage display method, a Nunc-immunotube (NUNC, cat No.4-442-2) was coated with 10ug of modified protective antigen (MPV) as the *Bacillus anthracis* protective antigen, and left at 37 °C for 2 hours with 4 % skim milk powder. In this case, phages were pre-cultured by mixing 4 % skim milk powder and phage pool at a ratio of 1:1 to remove non-specific phages. After the phage incubation, the pre-cultured phage was placed in the MPV-coated Nunc-immunotube and reacted at room temperature for 2 hours. A washing process was repeated 5 times in a first panning using 1×phosphate buffered saline with Tween 20 (PBST) (second plaining: 10 times, third panning: 20 times). Last washing was carried out twice with 1×PBS to remove tween-20, a surfactant contained in 1 ×PBST. 100 mM of triethylamine was used for elution, and 2M of Tris base was used for neutralization. In the same manner, phage enzyme-linked immunosorbent assay (ELISA) was performed for the antigen by performing up to the third panning.

For ELISA, the Nunc-Immuno plate was coated with 400 ng/well of the antigen at 4 °C for 16 hours, washed three times with 1×PBST, and incubated with 4 % skim milk powder for 2 hours at room temperature. After washing three times with 1×PBST, the scFv library of the Bacillus anthracis protective antigen of the monkey, and the first panning phage, second panning phage, and third panning phage obtained through the phage display were added and reacted at 37 °C for 2 hours. After washing three times with 1×PBST, anti-M13-HRP conjugated antibody (ThermoFisher Scientific) diluted by 1/3,000 was added and reacted at room temperature for 1 hour. After washing again, a color was developed with hydrogen peroxide (H₂O₂) and tetramethylbenzidine (TMB), and after stopping the reaction with 2M sulfuric acid (H₂SO₄), absorbance was measured at 450 nm. As a result, it was found that the phage panned with the Bacillus anthracis protective antigen had specific affinity for each antigen.

### 2-2. Monoclonal antibody selection

The monoclonal antibody is an antibody that recognizes one epitope and has low cross-reactivity, high specificity, and high affinity. Therefore, the monoclonal antibody was selected to specifically detect the Bacillus anthracis protective antigen.

The technique used to select monoclonal antibodies is based on those known in the art. Specifically, for example, single colonies from the second and third panning phage pools having specific affinity for the Bacillus anthracis protective antigen were inoculated with 2×YT medium containing 2 % glucose in a 96-deep well (Bioneer, 90030) and incubated at 37°C for 4 hours. After infecting a helper phage, the mixture was left at 37 °C for 30 minutes and shaken at 37 °C for 30 minutes. Thereafter, the culture medium was centrifuged at 5,000 rpm for 10 minutes to remove the supernatant, suspended in a new 2×YT medium containing magnesium chloride (MgCl₂), kanamycin, and carbenicillin, and cultured at 30 °C for 16 hours. After centrifuging the culture solution at 5,000 rpm for 10 minutes, the supernatant was subjected to ELISA in the same manner as above. As a result, 7 different clones of the Bacillus anthracis protective antigen antibody selected through antigenic function and sequencing analysis.

### Example 3. scFv-Fc expression and activity measurement

In Example 3, scFv-Fc was expressed as an antibody that specifically binds to the *Bacillus anthracis* protective antigen selected in Example 2, and the binding ability of the expressed scFv-Fc to the *Bacillus anthracis* protective antigen was confirmed by the following process.

### 3-1. Preparation of expression plasmids for scFv-Fc expression

Expression vectors were prepared for the 7 types of the clones of the *Bacillus anthracis* protective antigen antibody selected according to Example 2 in order to prepare antibodies in the form of fragment scFv-Fc having binding ability to the *Bacillus anthracis* protective antigen. Specifically, the scFv fragment of the *Bacillus anthracis* protective antigen inserted into the pComb3 vector was digested using the Sfi I restriction enzyme and then purified, and the pCEP-WPRE-Fc vector was digested using the same restriction enzyme and then purified. Then, a scFv-Fc vector was prepared by inserting the purified scFv fragment into the vector.

### 3-2. scFv-Fc expression and quantification

The prepared scFv-Fc vector was introduced into host cells and transformed, and the transformed host cells were cultured to express scFv-Fc, and an expression level of the scFv-Fc was quantified by ELISA.

First, the host cells, HEK293E cells, were inoculated at a concentration of 4×10⁶ cells/100 mm the day before transformation of the host cells. After mixing the DNA solution obtained by diluting 20 ug of DNA of the prepared scFv-Fc vector in 0.5 ml of 150 mM sodium chloride (NaCl) solution (pH5.5), and the PEI solution obtained by diluting 40 ug of polyethyenimine (PEI, Polyscience, Cat# 23966) in 0.5 ml of 150 mM sodium chloride solution (pH5.5) well, the mixture was left at room temperature for 10 minutes to prepare a DNA-PEI mixture. The DNA-PEI mixture was slowly added to the HEK293E cells cultured the previous day. After 6 days of transformation, the cell culture medium was collected, and a fresh medium was added again to culture for another 3 days. The culture medium was collected on the 9th day and the scFv-Fc expression was quantified by ELISA.

Specifically, to quantify the scFv-Fc expression, anti-human IgG Fc antibody diluted by 1/2,000 with PBS buffer was coated on a 96-well plate and left at 4 °C overnight. After washing three times with 1×PBST, it was reacted for 2 hours at room temperature with 2 % skim milk powder. After washing three times with 1×PBST again, the culture medium was added. In this case, the culture solution was diluted up to 10 times, 100 times, 200 times, 400 times, 800 times, 1,600 times, 3,200 times and 6,400 times. In the case of a standard product, a culture medium and human IgG as a standard product were added, reacted for 1 hour at room temperature, and washed three times with 1×PBST. The anti-human IgG HRP antibody was diluted by 1/2,500 and reacted at room temperature for 1 hour.

After the incubation was completed, the culture was washed three times with 1×PBST, developed color with tetramethylbenzidine (TMB) and hydrogen peroxide (H₂O₂) for 5 minutes. The reaction was stopped with 2N sulfuric acid (H₂SO₄), and absorbance was measured at 450 nm to determine the expression level of the scFv-Fc.

### 3-3. scFv-Fc purification

After culturing the HEK293E cells, which are host cells transformed by the same method as suggested in 3-2 above, the expressed scFv-Fc was isolated and purified from the cell culture medium by Biologics LP system (Bio-Rad) and Protein A agarose 6 fast flow.

First, a column was prepared by washing with a 0.5 N sodium hydroxide (NaOH) solution, and 1 ml of Protein A resin was added to the column, followed by washing with 1×PBS. The culture medium of the transformed host cells was passed down the column at a rate of 1 ml/min. To remove endotoxin, the column was washed with 100 ml volume of 1×PBS containing 0.1 % Triton-X 114 (polyethylene glycol p-(1,1,3,3-tetramethylbutyl)-phenyl ether). To remove residual Triton-X 114, the column was washed with 50 ml of 1×PBS and eluted with 0.1 M glycine (pH 3.0) as an elution buffer. After receiving 10 eluents of 3ml each, 1M Tris of pH 8.0 was immediately added in a volume of 1/10 of the elution buffer to neutralize the elution solution.

### 3-4. Analysis of scFv-Fc binding to the Bacillus anthracis protective antigen

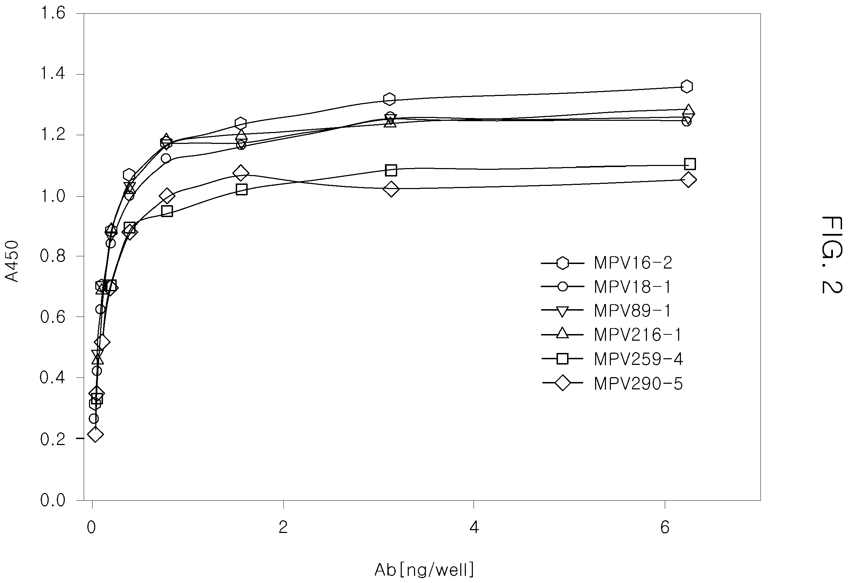

An antigen binding assay was performed to confirm whether the scFv-Fc expressed as an antibody bound to the Bacillus anthracis protective antigen. The Bacillus anthracis protective antigen was coated on a 96-well plate at 400 ng/well and left overnight at 4 °C. After washing three times with 1×PBST, it was reacted for 2 hours at room temperature with 2 % skim milk powder. After washing three times with 1×PBST again, the culture medium was added to the plate. After reacting at room temperature for 1 hour, the plate was washed 3 times with 1×PBST. The anti-human IgG-HRP antibody diluted by 1/2,500 was added to the plate and reacted at room temperature for 1 hour. After washing three times with 1×PBST, color development was performed with tetramethylbenzidine (TMB) and hydrogen peroxide (H₂O₂) for 5 minutes, the reaction was stopped with 2N sulfuric acid (H₂SO₄), and the absorbance was measured at 450 nm to determine the binding ability to the Bacillus anthracis protective antigen. The results are shown in FIG. 2.

FIG. 2 is a result of analyzing the binding ability of the expressed scFv-Fc to the Bacillus anthracis protective antigen. As shown in FIG. 2, it can be confirmed that all of the scFv-Fc expressed according to this example have excellent binding ability to the Bacillus anthracis protective antigen.

### Example 4. Preparation of humanized antibody

In general, it is known that humanized antibodies with high affinity can only be obtained by preparing more than 20 variants through CDR grafting, structural modeling, mutant preparation, antibody expression, antigen binding, affinity tests, etc. However, in the present invention, an antibody library of a smaller size was prepared, a monkey/human wobble sequence was introduced and randomly extracted, and then, an antibody specific to the Bacillus anthracis protective antigen having high affinity was isolated through panning..

Instead of selecting highly homologous human germ line sequences commonly used for framework regions for the preparation of an antibody library, a Herceptin framework, which is known to have been used clinically for a long time, has low immunogenicity, and is structurally very stable, was selected. A region defined by Kabat numbering was selected as a complementarity-determining region (CDR). As used herein, the term "complementarity-determining region (CDR)" refers to a region that imparts antigen-binding specificity among variable domains of an antibody.

As a result of comparing the Bacillus anthracis protective antigen with the human Herceptin framework, in the case of a heavy chain, it was considered to replace the amino acids at four places in the framework region with the sequences of Herceptin and monkey, and it was confirmed that the light chain showed very high homology. Therefore, the diversity of the humanized antibody library of the Bacillus anthracis protective antigen after the final design showed 2 light chains and 48 heavy chains, that is, the total library size of 96. These results are considered to be due to the high degree of homology between monkeys and human.

### 4-1. Preparation of humanized antibody scFv library

In order to create a scFv library for preparing a humanized antibody specific to the *Bacillus anthracis* protective antigen, the synthesis of heavy and light chains was performed through an overlap extension PCR method. The PCR was performed using the heavy and light chain synthetic primers in Tables 3 and 4 below, and a monkey monoclonal protective antigen scFv, which is the antibody fragment containing one or more complementarity-determining regions (CDRs), as a template.

The PCR was performed in the following way. First, in the case of the light chain, the overlap PCR was performed using 4 primers containing the part to be modified, and in the case of the heavy chain, the PCR was performed using 24 overlapping primers. As a result of the PCR, the variable domains of the heavy and light chains and the linker were obtained, and whole scFv fragments were prepared through three-fragment concatenation PCR using each fragment as a template.

In order to insert the synthesized scFv fragment of the library into the pComb3xss vector, it was treated at 50 °C for 16 hours using the Sfi I restriction enzyme, the scFv fragment was digested with the same restriction enzyme, and each was electrophoresed on agarose gel for purification, so that pure isolated vector and scFv fragments were obtained for insertion. After insertion, a humanized antibody scFv library was prepared by transforming the electrocompetent cells for transformation, E.coli ER2738 cell line.

Table 3 below shows the primer base sequences used for light chain synthesis.

**[Table 3]**

| Name | Sequence (5'-3') |
|---|---|
| MPV-89LF1 | GGGCCCAGGCGGCCGACATCCAGATGACCCAG |
| MPV-89LR1 | GGTGACCCGGTCTCCTACAGATGCAGA |
| MPV-89LF2 | GGAGACCGGGTCACCATCACTTGCCGG |
| MPV-89LR2 | CCCAGACCYACTGCCGCTGAACCT |
| MPV-89LF3 | GGCAGTRGGTCTGGGACAGATTTC |
| MPV-89LR3 | GTCGCGAAATCTTCAGGCTGCAGGCT |
| MPV-89LF4 | GAAGATTTCGCGACTTATTACTGTCAGC |
| MPV-89LR4 | TCGTTTGATCTCCACTTTGGT |

Table 4 below shows the primer base sequences used for heavy chain synthesis.

**[Table 4]**

| Name | Sequence (5'-3') |
|---|---|
| MPV-89H1 | GAAGTGCAGCTGGTG |
| MPV-89H2 | TCCACCGCCAGACTCCACCAGCTGCACTTC |
| MPV-89H3 | AGTCTGGCGGTGGACTCGTCCAACCTGGGG |
| MPV-89H4 | GAGAGACGCAGTGAGCCCCCAGGTTGGACG |
| MPV-89H5 | CTCACTGCGTCTCTCCTGCGCTGTGTCTGG |
| MPV-89H6 | ACCGCTGATGGAGCCACCAGACACAGCGCA |
| MPV-89H7 | GCTCCATCAGCGGTTACTGGTGGGGCTGGA |
| MPV-89H8 | GCCGGGGGSCTGCCTGATCCAGCCCCACCA |
| MPV-89H9 | AGSCCCCCGGCAAGGGCCTGGAGTGGATTG |
| MPV-89H10 | TGCCACCGATACACCCAATCCACTCCAGGC |
| MPV-89H11 | GGTGTATCGGTGGCAGTTCCGGGACCACCT |
| MPV-89H12 | GAGGGAGGGGTTATAGTAGGTGGTCCCGGAA |
| MPV-89H13 | CTATAACCCCTCCCTCAAGAGTCGAKTCACCA |
| MPV-89H14 | ACGTGTCCRYTGAAATGGTGACTCGACTCTT |
| MPV-89H15 | TTTCARYGGACACGTCCAAGAACACAGCCTA |
| MPV-89H16 | AGAGTTCATCTGCAGGTAGRMTGTGTTCTTGG |
| MPV-89H17 | CCTGCAGATGAACTCTCTGCGTGCCGAAGA |
| MPV-89H18 | GTAATACACGGCGGTATCTTCGGCACGCAG |
| MPV-89H19 | ACCGCCGTGTATTACTGTGCGAGAGGGGG |
| MPV-89H20 | AGAGTAATAACTACAACTAAAGCCCCCTCTCGCACA |
| MPV-89H21 | CTTTAGTTGTAGTTATTACTCTAACTGGGGACAGGG |
| MPV-89H22 | CACTGTGACCAGAGTTCCCTGTCCCCAGTT |
| MPV-89H23 | AACTCTGGTCACAGTGTCCTCAGGCCAGGC |
| MPV-89H24 | CTGGCCGGCCTGGCCTGAG |
| MPV-89Link-F | CCAAAGTGGAGATCAAACGAGGTGGTTCCTCTAGATCTTCCT |
| MPV-89Link-R | AGACTCCACCAGCTGCACTTCCCCACCACCGCCCGA |
| MPV-89AMP-F | GAGGAGGAGGAGGAGGCGGGGCCCAGGCGGCCGACAT |
| MPV-89AMP-R | GAGGAGGAGGAGGAGCCTGGCCGGCCTGGCCTGAGGAG |

### 4-2. Selection of humanized antibodies specific to the Bacillus anthracis protective antigen

Since the size of the humanized antibody scFv library for preparing a humanized antibody specific to the Bacillus anthracis protective antigen is small, soluble scFv was directly expressed without the need to use a phage display method, and the humanized antibody specific to the Bacillus anthracis protective antigen were selected by using the ELISA method using the Bacillus anthracis protective antigen.

First, 48 transformed colonies were inoculated into 1 ml of 2×YT medium containing 2 % glucose and 100 ug/ml of ampicillin, incubated overnight at 37 °C at 250 rpm, then re-inoculated into 2 × YT medium containing 100 ug/ml of ampicillin at a ratio of 1/100 and cultured from the optical density (OD) of 600 to 0.5. After incubation, 1 mM of isopropylβ-D-1-thiogalactopyranoside (IPTG) was added to the culture, followed by incubation at 30 °C overnight, and the culture medium was centrifuged at 5,000 rpm for 10 minutes to prepare a supernatant of soluble scFv.

For screening of the prepared scFv, ELISA was performed as follows. First, the protective antigen and bovine serum albumin (BSA) were dispensed at 200 ng/well on a maxisop 96 well plate manufactured by NUNC the day before screening, and coated overnight at 4°C. The next day, the coating liquid was removed, reacted at room temperature for 2 hours using 200 ul of 2 % skim milk powder, and washed three times with 300 ul of 1×PBST. 100 ul of scFv supernatant was added, reacted again at room temperature for 1 hour, washed again with 1×PBST 3 times. After adding 100 ul of anti-HA-HRP antibody diluted by 1000 times, the culture was reacted for 1 hour at room temperature. After the washing process was performed again, 100 ul of a TMB solution in which tetramethylbenzidine (TMB) and hydrogen peroxide (H₂O₂) were mixed was added for color development, followed by color development for 5 minutes. After 5 minutes, the reaction was stopped using 1.5N sulfuric acid (H₂SO₄), and absorbance was measured at 450 nm to select 17 clones that did not react to BSA but specifically reacted to the Bacillus anthracis protective antigen. By identifying the gene sequences of the selected 17 clones, it was confirmed that the substitution was made with the amino acid at the site to be substituted.

### 4-3. Manufacture of a humanized antibody IgG of the Bacillus anthracis protective antigen

In the case of the scFv fragment antibody, quantitative analysis is not possible, and conversion to IgG is required to confirm the neutralization capability. Therefore, the 5 clones showing high binding ability to the Bacillus anthracis protective antigen were converted into IgG and cloned into animal cell expression vectors.

In the case of the heavy chain of the selected 5 scFv fragment antibody clones, since the sequences are identical in some types, the same heavy chain primer was used for the same sequence, and PCR was performed using the primers shown in Table 5 below. Specifically, the scFv of each clone was used as a template for the heavy chain. 1 ul of the template gene, 1 ul of each primer, 5 ul of 10x pfu-X buffer, 1 ul of 10 mM dNTP mix, 0.5 ul of pfu-X polymerase, and distilled water were used to perform PCR (after performing 25 cycles at 95 °C for 5 minutes, at 95 °C for 30 seconds, at 58 °C for 40 seconds, and at 72 °C for 40 seconds, PCR was performed at 72°C for 10 minutes) to obtain a heavy chain fragment. The final PCR product was digested with BglII and NotI, ligated with the expression vector pCEP5 WPRE, transformed into *E. coli* DH5-a, and the DNA sequence of each clone was confirmed.

In the case of the light chain of the selected 5 scFv fragment antibody clones, the scFv of each clone was used as a template gene. 1 ul of template gene, 1 ul of primer, 5 ul of 10x pfu-X buffer, 1 ul of 10 mM dNTP mix, 0.5 ul of pfu-X polymerase, and distilled water were used to perform PCR (after performing 25 cycles at 95 °C for 5 minutes, at 95 °C for 30 seconds, at 58 °C for 40 seconds, and at 72 °C for 40 seconds, PCR was performed at 75°C for 10 minutes) to obtain a light chain fragment. The final PCR product was digested with BglII and BsiWI, and then inserted into the expression vector, pCEP5 WPRE. After insertion, *E. coli* DH5-a was transformed and the DNA sequence of each clone was confirmed.

Table 5 below shows the primer sequences used for whole IgG conversion of the heavy and light chains.

**[Table 5]**

| Name | Sequence (5'-3') |
|---|---|
| 89chiLF1 | CCATCTCGAGGCCACCATGTACTTGGGACTGAACTATGTATT C |
| 89ChiLR1 | ATCTGGATGTCACTCTGGACACCATTTAAGAG |
| 89ChiLF2 | GTCCAGAGTGACATCCAGATGACCCAGT |
| 89ChiLR2 | ATGATCGTACGTTTGATCTCCACTTTGGTCCC |
| 89ChiHR1 | AGCTGCACCTGACTCTGGACACCATTTAAGAG |
| 89ChiHF2 | TGTCCAGAGTCAGGTGCAGCTGGTGCAG |
| 89LhumF1 | GAGAAGATCTGCCACCATGTACTTGGGAC |
| 89LhumR1 | CATCTGGATGTCACTCTGGACACCATTTAAGAG |
| 89HhumF1 | GAGAGAATTCAGATCTGCCACCATGTACTTGGGACT |
| 89HhumR1 | GCTGCACTTCACTCTGGACACCATTTAAGAG |
| 89HhumF2 | GTGTCCAGAGTGAAGTGCAGCTGGTGGAGT |
| 89LhumR1 | CATCTGGATGTC ACTCTGGACACCATTTAAGAG |
| 89LhumF1 | GAGAAGATCTGCCACCATGTACTTGGGAC |
| 89LhumF2 | GGTGTCCAGAGTGACATCCAGATGACCCAGTCT |
| 89LhumR2 | ATGATCGTACGTTTGATCTCCACTTTGGTCCC |
| 89HChiTF1 | GAGAGAATTCAGATCTGCCACCATGTACTTGGGACT |
| 89HChiTR1 | AGCTGCACCTGACTCTGGACACCATTTAAGAG |
| 89HChiTF2 | GTGTCCAGAGTCAGGTGCAGCTGGTGC |

### 4-4. Expression and purification of a humanized antibody IgG of the Bacillus anthracis protective antigen

To do this, sub-cloning of the heavy and light chains of the antibody inserted into the pCEP5 WPRE was performed using the CHO-expressing cell line, dihydrofolate reductase (DHFR) (ATCC).

For sub-cloning of the modified expression vector pYAB (One vector system), the ligation enzyme for the heavy chain was changed to AvrII and BstZ17I. The light chain was similarly prepared by changing the ligation enzyme to EcoRV and PacI. The sub-cloning was performed in the order of heavy chain and light chain. After digesting the pYAB vector and the heavy chain with the restriction enzymes, AvrII and BstZ17I and ligating overnight at 4 °C, E. coli DH5-a was transformed and the DNA sequence was confirmed. The sequence-confirmed expression vector and light chain were digested with restriction enzymes, EcoRV and PacI in the same manner, ligated overnight at 4 °C, and then transformed into E. coli DH5-a, and the DNA sequence was confirmed.

The completed IgG expression vector was inserted into a CHO cell line using a transfection reagent (Freestyle MAX, Invitrogen). To prepare a cell line into which the expression vector was inserted, frozen CHO cells were passaged using CD-Forti CHO medium. After stabilizing the CHO cells by subculturing for 3 to 5 passages, they were inoculated into 125ml flasks at 0.5×10⁶ cells/ml the day before transfection. After 24 hours, the IgG expression vector was transformed by 1 cut digested with NruI. Transformation was performed using freestyle MAX. 50 ug of plasmid DNA and 50ul of freestyle MAX reagent were mixed in CD-Forti CHO medium to be stabilized to be 1.5ml each, and then mixed slowly to form a DNA-lipid complex and left at room temperature for 10 minutes. While the DNA-lipid complex was forming, the number of CHO cells was measured in the flask inoculated the previous day, and the cell number was adjusted to 1.0 × 10⁶ cells/ml to make 30 ml of the complex, and the DNA-lipid complex was dropped on the cells. After 48 hours, in order to improve the expression level of IgG and to select the CHO cell line into which the expression vector was inserted, methotrexate (MTX) and puromycin were treated to perform a total of two rounds of selection.

The first selection process for selecting the CHO cell line into which the expression vector was inserted was performed in a T-150 flask under conditions of treatment with 10ug/ml of 100nM puromycin (Gibco, A1113802) and 20u/ml of 200nM MTX (Sigma, A6770). The transfected cells were inoculated into a T-150 flask to have a concentration of 0.5×10⁶ cells/mix 10ml, and puromycin and methotrexate (MTX) were added under the above conditions. The T-150 flask was stationarily cultured at 37 °C, 8 % CO₂, and a viable cell count (VCD) was performed after 7 days. When the viable cell count was less than 30 %, the viable cell count was measured every 3 to 4 days, and when the viable cell count exceeded 30 %, the cells were transferred to a 125ml shaker flask (37 °C, 8 % CO₂, 150 rpm). In the 125 ml shaker flask, cells were inoculated at 0.5×10⁶ cells/ml × 30 ml, and passages were performed while identifying viable cell counts every 3 to 4 days. Finally, when the viable cell count reached 1×10⁶ cells/ml and the viability was 85 % or higher, the first selection process was terminated.

Then, the second selection process was performed in a 126 ml shaker flask by applying 30 ug/ml of 500 nM puromycin and 50 ug/ml of 1,000 nM methotrexate (MTX) to the CHO cell pool into which the expression vector from the first selection process was inserted. The cells were inoculated at 0.5×10⁶ cells/ml × 30 ml, and passages were performed while identifying the viable cell count every 3 to 4 days. When the viability was over 90 %, the second selection was terminated and each stable pool was stocked with CD-Forti CHO medium mixed with 10 % DMSO.

The CHO cell pool into which the second selection was completed and the selected expression vector was inserted was subject to a single cell limited dilution in a 96-well plate to increase the number of cells cultured in a 12-well plate, a 6-well plate, and a 125 ml flask in that order (scale up), and more than 100 subclones were selected by first performing 5 day 6 well evaluation batch culture (5D6WP) in a 6-well plate. In 5D6WP, cells were inoculated at 0.5×10⁶ cells/ml×3ml in a 6-well plate, cultured for 5 days, and then IgG titer was measured using Cedex bio as a method of identifying the IgG titer. Clones were selected by ranking from the top clones in expression level.

The clones selected as the 5D6WP were further screened by a simple fed batch culture (SFB) in a 125ml shaker flask. In the SFB, the cells were inoculated in an amount of 0.5×10⁶ cells/ml×30 ml in a 125 ml shaker flask, and only glucose was fed to be 4 g/L, 4 g/L, and 6 g/L on the 3rd, 5th and 7th days of culture, and finally, on the 14th day of culture, the top 10 clones were selected by measuring the IgG titer.

A basal media optimization and feeder optimization were performed with the selected 10 clones to confirm the expression level in other media, and finally, the CD with the most stable expression confirmed in the basal culture medium was selected as Forti CHO. In addition, as a result of the fed batch culture based on this, the final clone (205-41-30) was selected as a clone with stable metabolite and cell viability and high IgG expression. In order to perform a mycoplasma contamination test on the final production cell line, the final selected clone stocked in liquid nitrogen was thawed and subcultured for 3 passages, and then the mycoplasma test was performed using an e-MycoVALiD mycoplasma PCR detection kit, and as a result of requesting an external institution to additionally assay whether the final clone was contaminated with mycoplasma, it was confirmed as negative.

The nucleic acids and amino acid sequences of the light and heavy chains of the selected final humanized antibody specific to the Bacillus anthracis protective antigen were analyzed.

As a result, the humanized antibody specific to the Bacillus anthracis protective antigen of the present invention includes a light chain consisting of the amino acid sequence represented by SEQ ID NO: 1 and a heavy chain consisting of the amino acid sequence represented by SEQ ID NO: 2. The nucleic acid encoding the light chain of SEQ ID NO: 1 is represented by the nucleotide sequence of SEQ ID NO: 3, and the nucleic acid encoding the heavy chain of SEQ ID NO: 2 is represented by the nucleotide sequence of SEQ ID NO: 4.

In the humanized antibody specific to the *Bacillus anthracis* protective antigen, the three complementarity-determining regions of the light chain are, in order from the amino terminal to the carboxyl terminal, the light chain CDR1 represented by the amino acid sequence of SEQ ID NO: 5, the light chain CDR2 represented by the amino acid sequence of SEQ ID NO: 6, and the light chain CDR3 represented by the amino acid sequence of SEQ ID NO: 7. The three complementarity-determining regions of the heavy chain are, in order from the amino terminal to the carboxyl terminal, the heavy chain CDR1 represented by the amino acid sequence of SEQ ID NO: 8, the heavy chain CDR2 represented by the amino acid sequence of SEQ ID NO: 9, and the heavy chain CDR3 represented by the amino acid sequence of SEQ ID NO: 10. In one antibody, these six complementarity-determining regions come together to form an antigen binding site.

For the nucleotide sequences encoding the light chain CDR1, light chain CDR2, and light chain CDR3 constituting the light chain, the light chain CDR1 is encoded by the nucleotide sequence of SEQ ID NO: 11, and the light chain CDR2 is encoded by the nucleotide sequence of SEQ ID NO: 12, and the light chain CDR3 is encoded by the nucleotide sequence of SEQ ID NO: 13.

For the nucleotide sequences encoding the heavy chain CDR1, heavy chain CDR2, and heavy chain CDR3 constituting the heavy chain, the heavy chain CDR1 is encoded by the nucleotide sequence of SEQ ID NO: 14, the heavy chain CDR2 is encoded by the nucleotide sequence of SEQ ID NO: 15, and the heavy chain CDR3 is encoded by the nucleotide sequence of SEQ ID NO: 16.

In order to examine the efficacy of the final humanized antibody specific to the Bacillus anthracis protective antigen through the above examples, the following experimental example was performed.

### Experimental Example 1. Affinity analysis for a humanized antibody specific to the Bacillus anthracis protective antigen

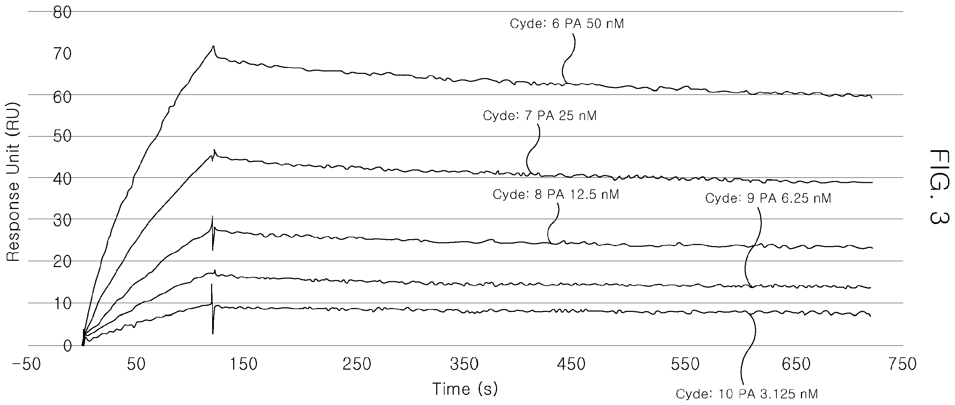

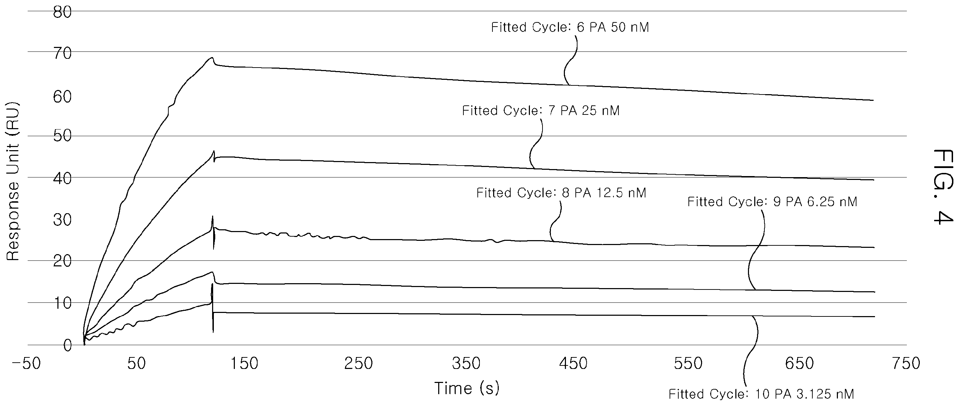

In order to measure the exact antigen binding affinity for each humanized antibody specific to the Bacillus anthracis protective antigen, a surface plasmon resonance technique was performed using a Biacore X-100 equipment. The humanized antibody specific to the Bacillus anthracis protective antigen was immobilized on a CM5 chip in a 10 mM sodium acetate solution at pH 5.5, and then regenerated in glycine pH 1.5 using the Bacillus anthracis protective antigen as an analyte. Then the binding force was measured. The concentrations of the Bacillus anthracis protective antigen used in the measurement were 3.125 nM, 6.25 nM, 12.5 nM, 25 nM, and 50 nM. In this case, the measured affinity results for the Bacillus anthracis protective antigen are shown in FIGS. 3 and 4, and the equilibrium dissociation constant (KD) value calculated for the Bacillus anthracis protective antigen based on FIGS. 3 and 4 was 9.37×10⁻¹⁰, which indicated the affinity for the antigen.

### Experimental Example 2. Analysis of toxin neutralization capability at a cellular level for a humanized antibody specific to the Bacillus anthracis protective antigen

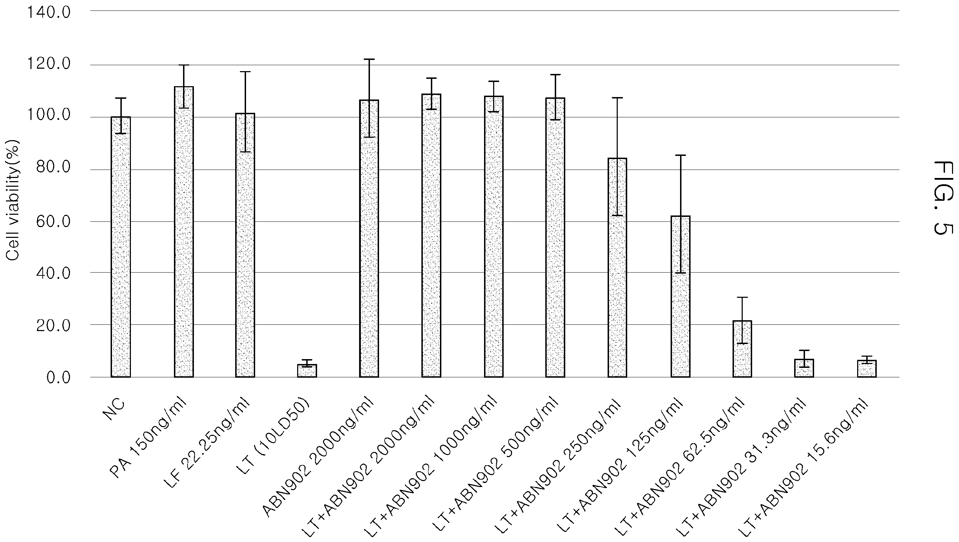

The toxin neutralization capability of the humanized antibody specific to the Bacillus anthracis protective antigen, which was selected for the Bacillus anthracis protective agent (PA), was confirmed using a mouse macrophage cell line, J774A.1. 1×10⁵ cell lines were put into a 96-well cell culture plate and cultured for 18 hours. As shown in FIG. 4, the selected humanized antibody (ABN902) specific to the Bacillus anthracis protective antigen was treated at concentrations of 2,000 ng/ml, 1,000 ng/ml, 500 ng/ml, 250 ng/ml, 62.5 ng/ml, 31.3 ng/ml, and 15.6 ng/ml. Lethal toxin (LT) is calculated as a cell lethal concentration of 10LD₅₀. A mixed solution was prepared by adding the Bacillus anthracis protective antigen (PA) and the lethal factor (LF) to final concentrations of 150 ng/mL and 22.25 ng/mL, respectively. This mixed solution was reacted in an incubator at 37° C for 1 hour, and then added to a cell culture plate. After the treated plate was incubated in the incubator at 37°C for 4 hours, water soluble tetrazolium (WST) reagent was added as to be 11.1 ul/well, and cultured in the incubator at 37°C for 2 hours. Thereafter, the absorbance was measured at a wavelength of 450 nm with a multi plate reader to confirm the viability of the cells against the antibody, and the results are shown in FIG. 5.

As shown in FIG. 5, it was confirmed that most of the cells were killed in LT (10LD₅₀) co-treated with the Bacillus anthracis protective antigen (PA) and the lethal factor (LF), and the cells survived normally in a control group (NC), the group treated with only 150 ng/ml of the Bacillus anthracis protective antigen (PA) or 22.25 ng/ml of lethal factor (LF). When treated with 500 ng/ml of the humanized antibody specific to the Bacillus anthracis protective antigen together with LT (10LD₅₀), the cells were not killed, confirming the toxin neutralization capability of the humanized antibody specific to the Bacillus anthracis protective antigen. However, it can be seen that the cells are killed when the treatment concentration of the humanized antibody specific to the Bacillus anthracis protective antigen is lowered to 250 ng/ml or less.

### Experimental Example 3. In vivo neutralization capability analysis using F344 rats

The in vivo neutralization capability of the humanized antibody specific to the *Bacillus anthracis* protective antigen was confirmed using F344 rats as an animal model. The *Bacillus anthracis* protective antigen (PA) and the lethal factor (LF) act as lethal factors in vivo and cause mortality. In rats, the half-lethal dose (LD₅₀) was calculated when the *Bacillus anthracis* protective antigen (PA) and the LF were administered intravenously at 12.3ug/kg and 4.1ug/kg, respectively.

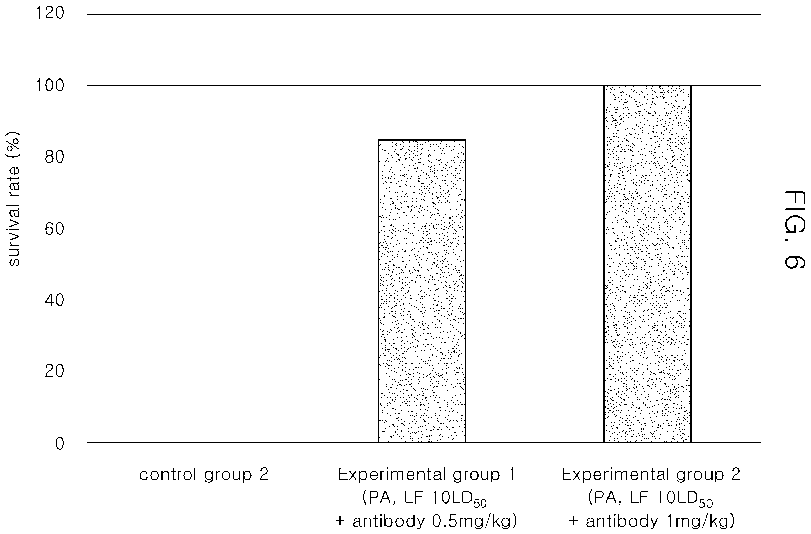

Using the calculated half-lethal dose, it was confirmed whether the humanized antibody specific to the Bacillus anthracis protective antigen could protect against 10LD₅₀ treated with the Bacillus anthracis protective antigen (PA) and the lethal factor (LF) in rats, and the results are shown in FIG. 6. As shown in FIG. 6, when 10LD₅₀ (PA123ug/kg, LF41ug/kg) treated with the Bacillus anthracis protective antigen (PA) and the lethal factor (LF) was administered intravenously to 3 F344 rats as control animals, all individuals died within 24 hours. On the other hand, in the case of Experimental Group 1, in which 0.5 mg/kg of a protective antigen antibody was intravenously administered and then the Bacillus anthracis protective antigen (PA) and the lethal factor (LF) were administered at 10LD₅₀, a survival rate was 85 %. 100% survival was confirmed in the case of Experimental Group 2, in which the Bacillus anthracis protective antigen (PA) and the lethal factor (LF) were administered at 10LD₅₀ after intravenous administration of 1 mg/kg of the antibody by increasing antibody dose.

### Experimental Example 4. In vivo neutralization capability analysis using rabbits

The in vivo neutralization capability of the humanized antibody specific to the Bacillus anthracis protective antigen was confirmed using rabbits. In the neutralization capability test with the rabbit, animal evaluation was performed using the Bacillus anthracis. The Bacillus anthracis induces death by expressing the Bacillus anthracis protective antigen (PA) and the lethal factor (LF) during internal infection. In rabbits, the half-lethal dose (LD₅₀) was calculated when 100 CFU/head of Bacillus anthracis spores was administered. Using the calculated half-lethal dose, it was confirmed whether the antibody had a protective effect against the Bacillus anthracis. The protective antigen antibody was administered intravenously at 100 mg/head, and the Bacillus anthracis strain was intramuscularly administered by dose (3 numbers/dose), and survival was confirmed for 14 days. When the Bacillus anthracis 10LD₅₀ was intramuscularly administered to 3 control animals, all of them died on the 2nd day. On the other hand, when the Bacillus anthracis was intramuscularly administered by dose after intravenous administration of 100 mg/head of the protective antigen antibody, all individuals survived up to a dose of 100LD₅₀. When the Bacillus anthracis was treated with 200LD₅₀, one of the three specimens died, showing a protective effect of about 67 %.

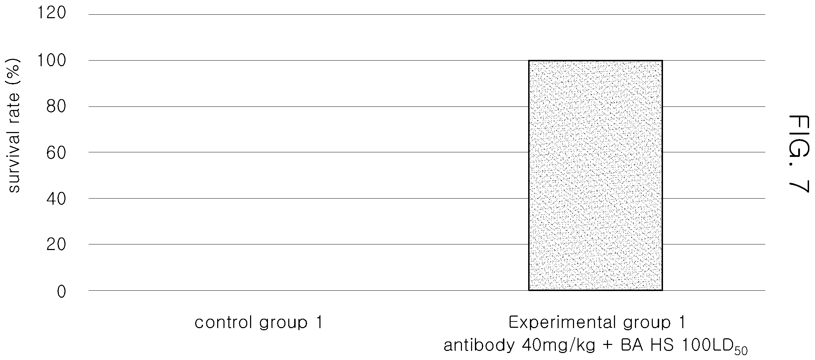

As a secondary test, a post exposure test was conducted in which the antibody was administered at 100 mg/head 24 hours after the Bacillus anthracis strain was administered at 50LD₅₀. As a result, as shown in FIG. 7, 100 % survival rate was shown in which all 4 out of 4 animals of Experimental Group 1 survived.

As reviewed in the above described Examples and Experimental examples, the composition containing the humanized antibody specific to the Bacillus anthracis protective antigen according to the present invention can be used as a pharmaceutical composition such as a composition for neutralizing Bacillus anthracis toxin and a composition for treating and preventing anthrax.

The term "treatment", as used herein, refers to all activities that alleviate and beneficially affect disease symptoms caused by the Bacillus anthracis through the administration of a composition containing a humanized antibody specific to the Bacillus anthracis protective antigen according to the present antibody. In addition, the term "prevention", as used herein, refers to all activities that inhibit or delay the development of diseases induced by the Bacillus anthracis by administering a composition containing a humanized antibody specific to the Bacillus anthracis protective antigen according to the present antibody.

The above-described preferable embodiments are provided so that the present invention can be performed by those skilled in the art, and the above embodiments and the appended drawings are merely set forth to illustrate, but are not construed to limit the scope of the present invention. Accordingly, those skilled in the art will appreciate that various modifications, additions and substitutions are possible, without departing from the scope and spirit of the invention as disclosed in the accompanying claims.

## Claims

1. A humanized antibody specific to a *Bacillus anthracis* protective antigen comprising:
a light chain consisting of an amino acid sequence represented by SEQ ID NO: 1; and
a heavy chain consisting of an amino acid sequence represented by SEQ ID NO: 2.

2. The humanized antibody specific to a Bacillus anthracis protective antigen of claim 1, wherein a nucleic acid encoding the light chain is represented by a nucleotide sequence of SEQ ID NO: 3.

3. The humanized antibody specific to a Bacillus anthracis protective antigen of claim 1, wherein a nucleic acid encoding the heavy chain is represented by a nucleotide sequence of SEQ ID NO: 4.

4. The humanized antibody specific to a Bacillus anthracis protective antigen of claim 1, wherein the light chain comprises:
a light chain CDR1 represented by an amino acid sequence of SEQ ID NO: 5;
a light chain CDR2 represented by an amino acid sequence of SEQ ID NO: 6; and
a light chain CDR3 represented by an amino acid sequence of SEQ ID NO: 7.

5. The humanized antibody specific to a Bacillus anthracis protective antigen of claim 4, wherein the light chain CDR1 is encoded by a nucleotide sequence of SEQ ID NO: 11,
the light chain CDR2 is encoded by a nucleotide sequence of SEQ ID NO: 12,
the light chain CDR3 is encoded by a nucleotide sequence of SEQ ID NO: 13.

6. The humanized antibody specific to a Bacillus anthracis protective antigen of claim 1, wherein the heavy chain comprises:
a heavy chain CDR1 represented by an amino acid sequence of SEQ ID NO: 8;
a heavy chain CDR2 represented by an amino acid sequence of SEQ ID NO: 9; and
a heavy chain CDR3 represented by an amino acid sequence of SEQ ID NO: 10.

7. The humanized antibody specific to a Bacillus anthracis protective antigen of claim 6, wherein the heavy chain CDR1 is encoded by a nucleotide sequence of SEQ ID NO: 14,
the heavy chain CDR2 is encoded by a nucleotide sequence of SEQ ID NO: 15,
the heavy chain CDR3 is encoded by a nucleotide sequence of SEQ ID NO: 16.

8. A method for preparing a humanized antibody specific to a Bacillus anthracis protective antigen, comprising the steps of:
(a) preparing an antibody library by synthesizing an antibody to a Bacillus anthracis protective antigen;
(b) selecting a Bacillus anthracis protective antigen antibody clone as an antibody that specifically binds to the Bacillus anthracis protective antigen from the antibody library;
(c) expressing and purifying a Bacillus anthracis protective antigen monoclonal antibody by introducing an expression vector containing a gene of the Bacillus anthracis protective antigen antibody clone selected in step (b) into an antibody expressing host cell;
(d) preparing a humanized antibody library of the Bacillus anthracis protective antigen containing complementarity-determining regions of an antibody using the Bacillus anthracis protective antigen monoclonal antibody as a template;
(e) selecting a humanized antibody clone of the Bacillus anthracis protective antigen as an antibody specifically binding to the Bacillus anthracis protective antigen from the humanized antibody library of the Bacillus anthracis protective antigen; and
(f) preparing a humanized antibody specific to the Bacillus anthracis protective antigen by introducing an animal cell expression vector containing the gene of the humanized antibody clone of the Bacillus anthracis protective antigen selected through step (e) into the antibody expressing host cell.

9. The method of claim 8, wherein the (c) step comprises the steps of:
preparing a recombinant expression vector containing the gene of the Bacillus anthracis protective antigen antibody clone;
introducing the gene of the Bacillus anthracis protective antigen antibody clone into the antibody expressing host cell using the recombinant expression vector and transforming the host cell; and
purifying the antibody from a culture medium in which the transformed antibody expressing host cell is cultured.

10. The method of claim 8, wherein the (f) step comprises the steps of:
preparing a recombinant animal cell expression vector containing the gene of the humanized antibody clone of the Bacillus anthracis protective antigen from the humanized antibody library of the Bacillus anthracis protective antigen;
introducing the gene of the humanized antibody clone of the Bacillus anthracis protective antigen into the antibody expressing host cell using the recombinant animal cell expression vector and transforming the host cell;
purifying the humanized antibody specific to the Bacillus anthracis protective antigen from a culture medium in which the transformed antibody expressing host cell is cultured.

11. The method of claim 8, wherein in case that a complementarity-determining region of the antibody is a light chain complementarity-determining region of the antibody, the light chain complementarily-determining region of the antibody comprises:
a light chain CDR1 represented by an amino acid sequence of SEQ ID NO: 5;
a light chain CDR2 represented by an amino acid sequence of SEQ ID NO: 6; and
a light chain CDR3 represented by an amino acid sequence of SEQ ID NO: 7.

12. The method of claim 11, wherein the light chain CDR1 is encoded by a nucleotide sequence of SEQ ID NO: 11,
the light chain CDR2 is encoded by a nucleotide sequence of SEQ ID NO: 12,
the light chain CDR3 is encoded by a nucleotide sequence of SEQ ID NO: 13.

13. The method of claim 8, wherein in case that a complementarity-determining region of the antibody is a heavy chain complementarity-determining region of the antibody, the heavy chain complementarily-determining region of the antibody comprises:
a heavy chain CDR1 represented by an amino acid sequence of SEQ ID NO: 8;
a heavy chain CDR2 represented by an amino acid sequence of SEQ ID NO: 9; and
a heavy chain CDR3 represented by an amino acid sequence of SEQ ID NO: 10.

14. The method of claim 13, wherein the heavy chain CDR1 is encoded by a nucleotide sequence of SEQ ID NO: 14,
the heavy chain CDR2 is encoded by a nucleotide sequence of SEQ ID NO: 15,
the heavy chain CDR3 is encoded by a nucleotide sequence of SEQ ID NO: 16.

15. The method of claim 8, wherein the humanized antibody specific to the Bacillus anthracis protective antigen comprises:
a light chain consisting of an amino acid sequence represented by SEQ ID NO: 1; and
a heavy chain consisting of an amino acid sequence represented by SEQ ID NO: 2.

16. A composition for preventing and treating a disease caused by a Bacillus anthracis infection, comprising as an active ingredient the humanized antibody specific to the Bacillus anthracis protective antigen of claim 1.
